# EUROPEAN PATENT SPECIFICATION

(11) **EP 2 760 573 B1**
(45) Date of publication and mention of the grant of the patent: **19.12.2018**
(21) Application number: 12780407.8
(22) Date of filing: 28.09.2012
(51) Int. Cl.: B01F 3/08, B01F 5/00, B01F 5/02, B01F 5/04, B01F 5/10, C12M 1/00, C12M 1/33, C12M 1/107

(54) **GAS MIX INSTALLATION AND METHOD**
GASMISCHANLAGE UND VERFAHREN DAFÜR
INSTALLATION DE MÉLANGE DE GAZ ET PROCÉDÉ CORRESPONDANT

(30) Priority: 30.09.2011 DK 201170540
(43) Date of publication of application: 06.08.2014
(73) Proprietor: Landia A/S, 6940 Lem St (DK)
(72) Inventor: HJULMAND, Poul, DK-6940 Lem St (DK); TOBLER, Kurt, DK-6900 Skjern (DK); LARSEN, Steen Buhl, DK-6900 Skjern (DK)
(74) Representative: Tellefsen, Jens J.
(86) International application number: PCT/DK2012/050364
(87) International publication number: WO 2013/044926

(56) References cited:
- EP-A1- 0 563 434
- EP-A2- 0 801 229
- DE-U1- 8 524 206
- GB-A- 2 189 237
- US-A- 2 245 035
- US-A- 5 075 016
- US-A- 5 302 082
- US-A- 5 316 682
- US-A- 5 523 234
- US-A- 5 824 243

## Description

### Field of the Invention

The present invention relates to a gas mix installation for use in connection with tanks for biogas reactors and digesters, as well as a method of using said gas mix installation in connection with production of biogas.

### Background of the Invention

The production of biogas made from manure, sludge from wastewater and/or organic material is becoming increasingly popular, and due to more refined technologies the yield is becoming economically interesting. In the art there are a number of different constructions used in order to create the biogas where most of the methods include storing the basic material, i.e. the manure and/or organic waste in reactor tanks or storage tanks. In these tanks the basic material is left to ferment in that bacteria in the material or added with water to the material will decompose the basic materials and in the process create a substantial amount of gas. Usually the process is divided into two general types of processes called aerobic and anaerobic processes, i.e. with or without the presence or addition of extra oxygen.

The present invention is suitable for both aerobic and anaerobic processes, but is especially advantageous when used in the anaerobic processes.

Typically, biogas consists of approximately 60% methane (CH₄), 37% carbon dioxide (CO₂) and 3% miscellaneous gases including hydrogen sulphide (H₂S). In the process it is desirable to obtain as much pure methane (CH₄) as possible.

Further, as the manure used as basic material in biogas production comprises both liquid and substantially solid particles, it is necessary to stir the contents of a bioreactor tank in order to allow the bacteria to have access to all surfaces of the basic material in order to decompose the material which in turn produces the biogas. For these reasons various systems are provided in order to continuously stir the basic materials stored in the tanks in order to optimize and speed up the process of biogas production.

It is also known, see for example DE102008046615, to treat the basic material in order to increase the yield of biogas and reduce the reaction time in the reactor/digester. A widespread treatment is hydrolysis, where the hard to decompose components of the manure, such as cellulose, hemicellulose, pectin, and lignin (collectively known as lignocellulose) are treated. The hydrolysis process breaks down these components into simple monosaccharides, which the bacteria, more easily and faster convert to biogas. Typically the hydrolysis requires extra processes which in turn are energy consuming, having a negative influence on the extra yield obtained by the process. In DE102008046615 a special hydrolysis device is provided inside of which part of the basic material is specifically treated in order to hydrolyse this part of the material.

From GB2189237 is known a gas mixing installation comprising a pump connected to a reactor on the suction side via a pipe. A biogas pipe is connected on the injection side of the pump using a venture-like device. The pump and the biogas supply pipe are mounted on the external side of the reactor.

### Object of the Invention

It is an object of the present invention to further improve the prior art methods by providing improved mixing and processing of the organic materials in the basic materials increasing the biogas yield and speeding up the process, all at the same time.

### Description of the Invention

The present invention addresses this by providing a gas mix installation according to claim 1.

The invention provides a number of advantages by this installation such as by sucking out of the tank the material which is creating the biogas inside the tank circulating the material through a main pump where the substantially liquid base material inside the tank is ejected from the pump at a higher pressure whereby the injection of material inside the tank will create movement in the content of the tank and thereby provide the stirring action comparable to the stirring action created by vanes in the prior art. By simply stirring the material without having implements inside the tank and especially inside an anaerobic bioreactor it is not necessary to access the interior of the tank in order to service, maintain or repair the equipment, but all repairs may be carried out from the outside of the tank due to the construction of the gas mix installation where only the injection pipe and the suction pipe are connected to the interior of the tank.

By furthermore providing a gas pipe to the injector pipe the higher pressure in the liquid on the injection side of the pump will suck gas into the liquid stream and thereby mix it with the liquid to be injected into the tank. This will provide a much larger volume of injected liquid/gas into the tank such that the main pump's capacity is surpassed by the addition of gas whereby a larger volume will be put into motion inside the bioreactor tank. Furthermore, due to the buoyancy of the gas contained in the injected liquid the gas will percolate towards the top of the tank thereby in addition to creating a horizontal circulation in the liquid inside the tank about a vertical axis, the injected mixture will create circulation around a horizontal axis as well.

This three-dimensional turbulence in the liquid inside the bioreactor tank will ensure that the materials in the basic material used for decomposition and thereby generation of biogas is optimized at all times. Usually, the temperature will vary from the top of the tank to the bottom of the tank, but with arranging gas mix installations in appropriate places having a sufficient capacity it is possible to more or less eliminate the temperature profile through the liquid such that optimum process conditions will be present at substantially any level in the tank.

Furthermore, by rotating the liquid around a horizontal axis, i.e. creating extra turbulence by injecting gas in addition to the liquids, a further advantage is achieved. Traditionally, by stirrring the material in the tanks by means of vanes or the like a top layer of light materials will be created substantially covering the top surface of the liquid and thereby hampering the escape of the biogas from the liquid. The turbulence created by the bubbles will minimize or altogether eliminate this effect such that a free liquid surface will be present due to the bubbles escaping the surface creating the circulation around the horizontal axis.

It should in this context be considered that the basic material may be composed of manure from substantially any domestic animal and/or the manure may be provided with further organic material such as for example a grass, any type of cereals, ensiled crops such as for example corn and the like.

Furthermore, sewage or sludge from wastewater treatment plants, fats, bi-products from food processing plants, organic household waste or any other waste or sludge containing decomposable organic material may also be used as a basic material for the generation of biogas.

This embodiment with the shredder type pump in combination with the injector pipe creates a further improved biogas reaction.
The present invention in this embodiment in addition to creating the gas - liquid mixture also creates hydrolysis, without additives, special equipment and the like.

In a further advantageous embodiment of the invention the gas pipe's inlet in the injector pipe is in a venturi device arranged in the injector pipe. A venturi is a known physical construction where for example a liquid is passed through a nozzle into a space larger than the dimension of the nozzle whereby an under-pressure is created around the liquid jet which under-pressure in this embodiment of the invention is used to suck in the gas and mix it with the liquid jet. By dimensioning the venturi appropriately a large amount of gas may be sucked into the liquid jet and thereby injected into the bioreactor tank.

The Venturi effect is a jet effect; as with an (air) funnel, or a thumb on a garden hose, the velocity of the fluid increases as the cross sectional area decreases, with the static pressure correspondingly decreasing. According to the laws governing fluid dynamics, a fluid's velocity must *increase* as it passes through a constriction to satisfy the principle of continuity, while its pressure must *decrease* to satisfy the principle of conservation of mechanical energy. Thus any gain in kinetic energy a fluid may accrue due to its increased velocity through a constriction is negated by a drop in pressure. An equation for the drop in pressure due to the Venturi effect may be derived from a combination of Bernoulli's principle and the continuity equation.

In a still further advantageous embodiment of the invention the injector pipe upstream from the connection to the gas pipe is provided with a branch pipe, which branch pipe is provided with a secondary injector pipe. In this embodiment it is foreseen that the same pump may be used to provide liquid at relatively high pressure, both through an injector pipe connected to a gas pipe such that gas will be mixed with the liquid before it is injected back into the tank and to an injector which injects the liquid back into the tank at a higher pressure but without gas. This embodiment is especially advantageous in larger tanks or in tanks where relatively heavy basic materials are used in that the density of the liquid is higher than the density of the liquid mixed with gas such that in situations where it is needed to use larger force in order to rotate and thereby mix the basic materials inside the bioreactor the pure liquid injectors may advantageously be arranged in addition to the injectors mixing the liquid with gas. Both types of injector use the same pump.

The main pump is a shredder type pump comprising means for cutting, chopping or shredding any solids in the liquid. As already mentioned above this vastly increases the biogas production and at the same time decreases the process time such that more gas may be developed from the same amount of biological base material and faster. By shredding the solids in the basic material the bacteria will instantly have a much larger access surface to attack. At the same time the basic material inside the tank becomes more homogenous whereby a better mixing and turbulence in the tank is achieved such that even faster reaction times are obtained.

This embodiment with the shredder type pump in combination with the injector pipe comprising a venturi device in combination creates a further improved biogas reaction.

As the liquid passes the venturi device and is exposed to the relatively dramatic pressure change, part of the hydrogen sulphide and CO₂ will be decomposed/ripped apart, and a chemical transformation into SO₂ and CH₄ will occur. Tests with the method therefore indicate that the methane content of the biogas will typically increase from the 60% as mentioned above to more than 65% by going through the venturi. By further mixing and chopping any solids in the liquid and thereby creating a liquid containing finer particles, the pressure drop in the venturi is further increased and thereby the ripping of the hydrogen sulphide is increased. In the disintegrating process both in the shredder pump and the venturi additional carbon is released which comes from the interior of the cells which are ripped apart both during the shredding action, but also during the passage in the venturi due to the high difference in pressure. This process of cell destruction is also known as hydrolysis as already discussed in the introduction. The extra released carbon is used in the formation of CH₄ methane which is the desired gas in the biogas production process.

The present invention in this embodiment in addition to creating the gas - liquid mixture also creates hydrolysis, without additives, special equipment and the like.

In a further advantageous embodiment the diameter of the injector pipe at least on a section of said pipe has an interior diameter of 60-100 mm. Tests have indicated that for the typical tank sizes used in bioreactors there is an optimal size on the injector pipe which is a balance between how much liquid and how much gas is to be injected in the tank in order to create optimum reaction conditions for the bioreactor inside the tank. Typically having an interior diameter of 60-100 mm of the injector optimum reaction conditions will be achieved. It should, however, in this context be noted that also a host of other factors such as the contents in the tank the constituents of the basic material used for the bioreactor process, the size of the pump, the size of the tank, the temperature both inside and outside of the tank etc. have influence on these conditions. For example for normal tanks, i.e. tanks up to a diameter of approx 4-50 m the following relationships are relevant: using a 10 to 20 kW pump of the Landia Chopper type the diameter should be chosen between 60 to 70 mm, for 20kW to 30kW the diameter should be 70-85 mm and for 30 - 40kW pumps the diameter should be 85 - 100 mm. For larger tanks and/or larger pumps the diameter should be dimensioned accordingly, and furthermore the number of gas mix installations should also be adjusted.

In a further advantageous embodiment the suction pipe in use is arranged below the injection pipe. In this manner it is foreseen that the suction pipe does not draw gas containing liquid into the pump which could increase problems with cavitation etc. in the pump as such and thereby lower the effectiveness of the pump. Also by sucking up the liquid from the bottom of the tank, the vertical rotation of the material in the tank is further increased such that in addition to the turbulence created by the bubbles percolating towards the top of the tank this is further reinforced by the suction the suction pipe.

The injector pipe, at least the part of the pipe which in use projects into the tank, is arranged at an angle relative to the side of the wall of the tank, where said angle is selected between 15° and 40°; the embodiment where in use the injector pipe is arranged at an angle relative to the wall of the tank, which is different to the angle at which the suction pipe or at least the the part of the suction pipe which projects into the tank, is arranged relative to the wall of the tank, also provides significant advantages.

With these arrangements optimum circulation in three dimensions inside the tank is achieved.

The invention is also directed to a method of increasing the gas yield from a biogas reactor tank, wherein one or more gas mix installations as discussed above is/are installed at a predetermined distance from the bottom of the reactor tank;
where the injector pipes are arranged to inject a gas/liquid mix into the tank at an oblique angle relative to the wall of the reactor tank, whereby the liquid in the tank is stirred;
where the gas pipe uses the reservoir of biogas above the surface of the liquid, inside the tank as a source of gas, allowing the gas to percolate up through the liquid.

Especially the embodiment of the method where enzymes are introduced into the liquid before the liquid - gas mix is injected in the tank is advantageous. The mixing in the venturi device and the injection into the tank, creates a highly turbulent situation where the enzymes are effectively and substantially evenly distributed in the mixture. As the mixture is introduced into the tank the enzymes will be effectively mixed with the contents of the tank, and further improve the biogas production.

The advantages of the method, also as described in the further embodiments where the under-pressure in the suction pipes and the over-pressure in the injection pipe causes the liquid inside the tank to rotate in a horizontal plane and circulate in a vertical plane, and where a plurality of gas mix installations are arranged along the periphery of the bioreactor, in one or more levels relative to the tank's bottom, is already discussed above

### Description of the Drawing

The invention will now be explained with reference to the accompanying drawing wherein
- Figure 1: illustrates schematically the principles of the invention
- Figure 2: illustrates a plain view through a cross section of the tank
- Figure 3: illustrates a vertical cross-section of the flow
- Figure 4: illustrates an installation with a plurality of devices
- Figure 5: illustrates a close-up of a section of a tank wall with a gas mix installation
- Figure 6: illustrates a cross-section through an injection pipe
- Figure 7: illustrates the injection pipe
- Table 1: illustrates various comparative data relating to the process

### Detailed Description of the Invention

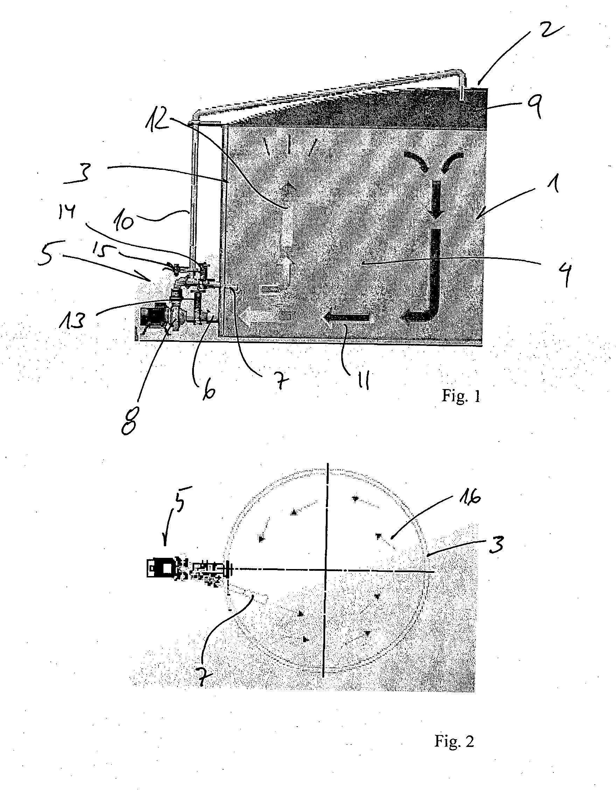

Generally schematically illustrated in fig 1 is a gas mix installation (5) for use in connection with tanks for biogas reactors (1) wherein the gas mix installation comprises:
- a main pump (8) having respectively a suction side and an injection side, where said pump (8) is arranged externally of the tank (1);
- a liquid suction pipe (6) connecting the interior of the tank (1) to the main pump's (8) suction side;
- an injector pipe (7) connecting the interior of the tank to the pump's injection side;
- a gas pipe (10) connected between the injector pipe (7) and a source of biogas (9); where said gas pipe (10) is connected to the injector pipe (7) externally of the tank (1) In figure 1 is schematically illustrated the principles of the invention. A biogas reactor 1 partly seen in cross-section is filled with a liquid containing basic material for the generation of biogas, mainly methane. The biogas reactor 1 is of the anaerobic type in that a lid 2 is provided on the reactor such that any biogas generated from the liquid 4 is collected at the top of the bioreactor. In a wall 3 of the bioreactor is arranged a gas mix installation 5 according to the present invention. The gas mix installation 5 comprises a suction pipe 6, an injector pipe 7 and a pump 8. The suction pipe 6 is connected to the suction side of the pump and the injector pipe 7 is connected to the ejector side of the pump 8. When the pump is activated liquid including basic material 4 will be sucked into the suction pipe 6 through the pump 8 and ejected back into the bioreactor tank 1 at a higher pressure. Between the pump 8 and the nozzle of the injector pipe 7 a gas pipe 10 is connected to the injection pipe 7. In this embodiment the gas pipe 10 is in its opposite end connected to the gas 9 generated inside the biogas reactor 1 such that the gas is recirculated from the gas reservoir 9 through the gas pipe 10 and the injector 7 towards the bottom of the liquid and basic material 4 contained inside the bioreactor tank 1. In this manner the same gas is circulated through the basic material and the liquid a number of times. It is, however, also contemplated that the gas source may be for example from an adjacent bioreactor tank at a different stage of the process or an altogether different gas source.

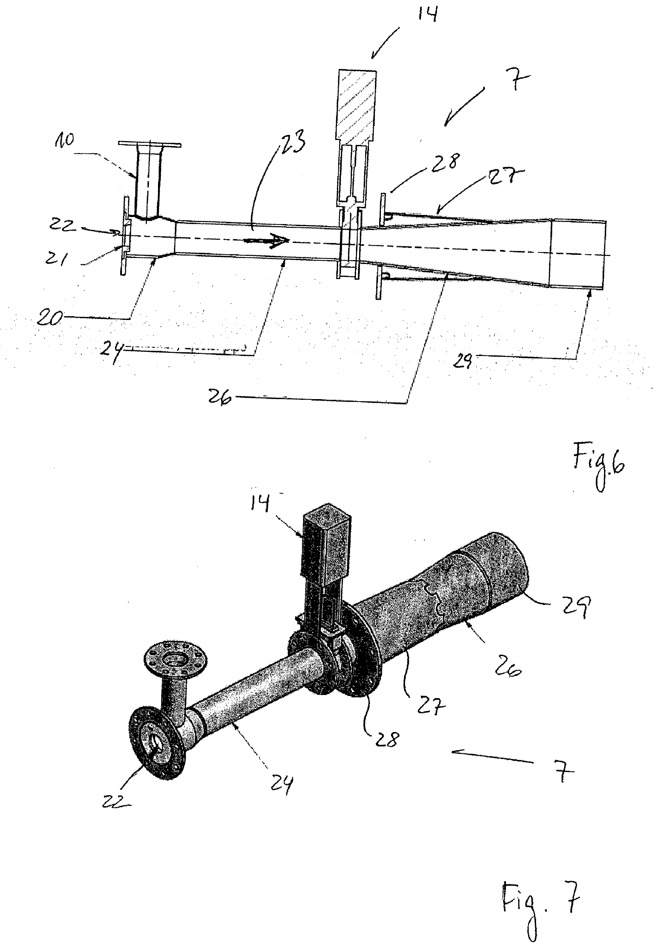

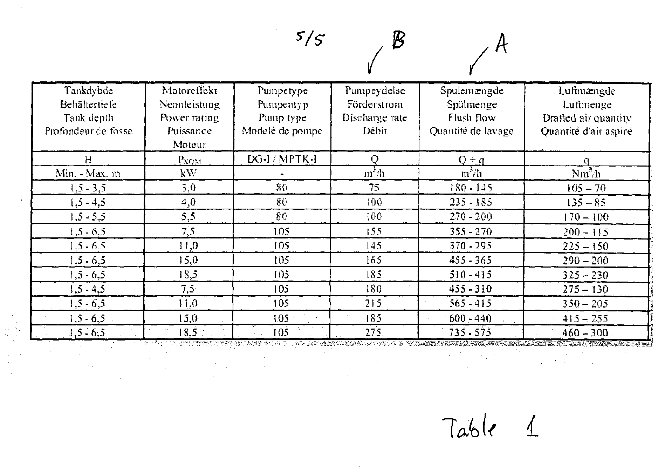

When the pump 8 is activated, it will create a current indicated by the arrow 11 in that the liquid and basic material kept inside the bioreactor will be sucked towards the suction pipe 6 due to the under-pressure created by the pump 8. The pump will generate a higher pressure and re-inject the liquid containing basic material into the bioreactor again. During the material's and the liquid's passage through the pump a venturi device which will described in more detail with respect to figure 6 will cause gas to be mixed with the high-pressure liquid before it is injected into the bioreactor tank. As the gas/liquid/material is introduced into the tank, the gas will tend to percolate and due to its buoyancy move towards the surface as indicated by the arrows 12.

In addition to the features of the gas mix installation already described above, the gas mix installation is furthermore provided with valves 13, 14, 15 making it possible to selectively cut off the suction pipe 6 by valve 13, the injection pipe 7 by valve 14 and/or the gas pipe 10 by valve 15. In this manner it becomes possible to service and replace any broken parts without having to empty and clean the tank before gaining access to the means necessary in order to create the flows indicated by the arrows 11, 12.

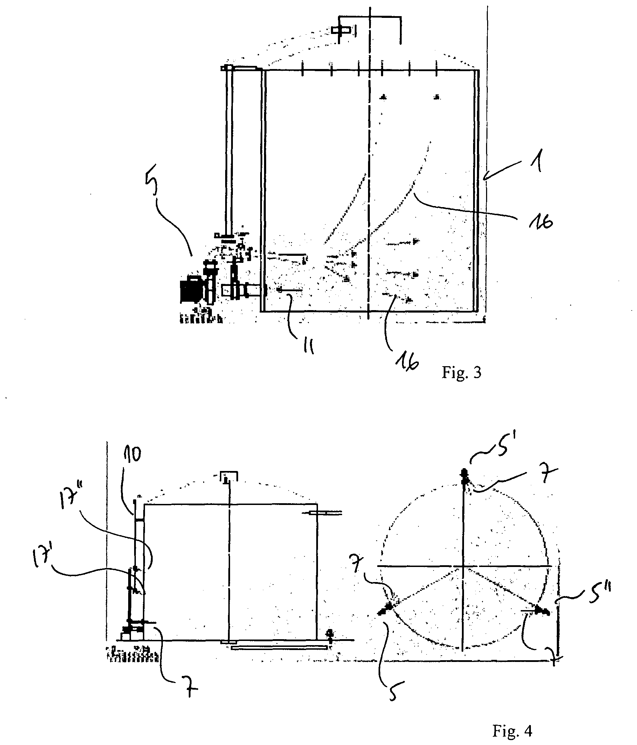

Turning to figure 2 a plain view through a cross section of the tank is illustrated where the arrows 16 indicate the flow in the tank created by the injection pipe 7. In this embodiment a single gas mix installation 5 is utilized in order to create circulation substantially in the entire tank, whereas for larger tank installations it will be necessary as indicated in figure 4 to use more installations. The flow seen from above in figure 2 is depicted in a vertical cross-section in figure 3, and it is clear that substantially the entire volume inside the tank 1 is being agitated by the installation 5. As illustrated by the arrows 16 in combination with the flow illustration from figure 2 it is evident that substantially the entire volume is being agitated by the installation 5.

For larger tanks, however, as illustrated with reference to figure 4 it may be necessary to install a plurality of devices, in this case three gas mix installations 5, 5', 5". The injector pipes 7 are arranged at an angle relative to the wall of the tank 3 such that a circular flow corresponding to the flow depicted and described with reference to figure 2, see arrow 16, will result.

On the left-hand portion of figure 4 is illustrated a cross-section through the tank where it may be seen that additional gas injectors 17', 17" are arranged connected to the gas pipe 10. In this manner due to the larger dimensions of the tank it becomes possible to effectively expose a large portion of the basic material in the liquid to the gas and thereby increase the yield of biogas.

The tank illustrated in fig 4 represents a large tank. In these tanks stirring or circulation of the contents of the tank, whether it be by means of the inventive gas mix device or by traditional vane means, will create a pile of solids, typically sand and the like in a centre part of the tank on the bottom. Over time this pile may have a significant volume, consequently limiting the effective volume of the tank. Therefore in these types of tanks a sediment evacuation system 30,31,32 may be provided. The system comprises a bottom valve 30, a pipe 31 and a pump 32.

In smaller tanks the rotation of the material inside the tank will minimise or altogether eliminate the build up of sediment.

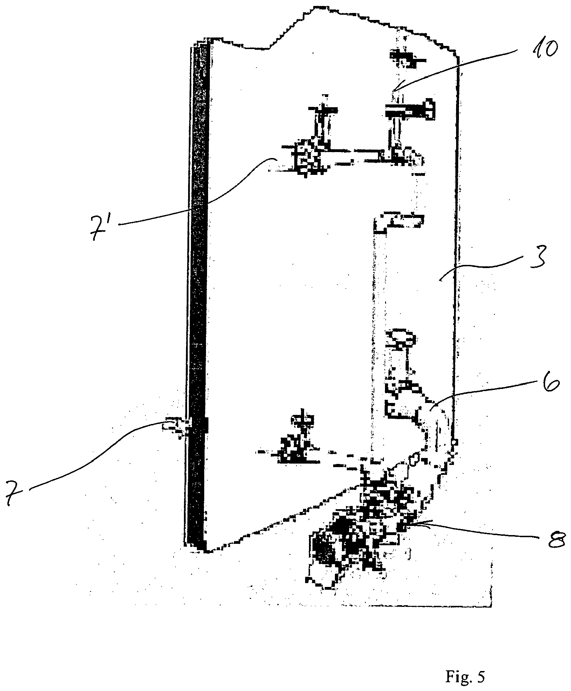

In figure 5 is illustrated a close-up of a section of a tank wall 3 in which a gas mix installation 5 according to the invention is arranged. The gas pipe 10 supplies gas for two injector 7'. The pump 8 may advantageously be of the chopper or shredder type available from Landia, Denmark. As the suction pipe 6 sucks liquid and basic material from the lower region of the tank into the pump 8, the shredders or choppers mounted in the pump 8 will chop up, mince and/or dissect any solid material in the liquid mixture extracted through the suction pipe from the tank. As this mixture is further pumped through the injection nozzle and mixed with gas, a more homogenous liquid containing relatively fine particles is injected into the liquid in the tank. This injection causes, due to the oblique arrangement of the injection nozzle 7' relative to the tank's wall 3 (typically between 5° and 25°), a rotation as illustrated in figure 2. The injection pipes 7' is provided with a venturi device as will be explained in more detail with reference to figure 6. The venturi device makes it possible to mix gas into the liquid containing finely chopped or minced particles in connection with the injection pipes 7'. The lower injection nozzle 7 does not include a venturi device, and therefore the liquid injected through nozzle 7 will only cause the basic material to be finely shredded due to its passage through the pump 8, and due to the inertia of the liquid injected, the liquid in the tank will be brought to rotate.

Furthermore, as the liquid containing chopped and/or minced particles passes through the venturi, the pressure difference will further disintegrate the particles, and even tear the cellular structure (hydrolysis). In this process further carbon is exposed/provided for the generation of CH₄. Consequently, both injection pipes 7, 7' will inject a mixture of liquid containing finally chopped or minced basic material usually stemming from organic waste as explained in the introductory part of the description and recycled biogas.

Turning to figure 6 a cross-section through an injection pipe 7 is illustrated. The gas pipe 10 is connected to a mixing chamber 20. Adjacent an upstream of the mixing chamber is arranged a nozzle 21 where the nozzle has an opening or orifice 22 which opening 22 is smaller than a cross-section perpendicular to the flow direction indicated by the arrow 23, such that as the chopped up liquid is forced from the pump through the injector pipe 7, a venturi effect will arise in the mixing chamber 20 due to the lower pressure to which the liquid by passage of the orifice 22 in the nozzle 21 is exposed. The lower pressure/under-pressure will suck gas from the gas pipe 10 into the liquid stream which will be mixed with gas through the ejector pipe 24. Following the ejector pipe is arranged a valve 14 which valve 14 may shut off the liquid connection between the interior of the tank and the exterior of the tank. The ejector pipe is connected to a transition pipe 26 which transition pipe in this embodiment has a conical cross-section such that the pressure in the liquid will diminish. Superposed the transition pipe 26 is a mounting pipe 27 which comprises flanges 28. In use the mounting pipe 27 will be inserted and correspond to the thickness of the wall of the tank and the diameter corresponds to the hole made in the wall of the tank, and the flanges 28 will be used to mount the injection pipe 7 to the wall of the tank. At the distal end of the injector pipe is arranged a defuser 29 which defuser 29 introduces the liquid mix with gas into the liquid contained in the tank and creates the turbulence/rotation in the liquid stored in the tank as described above.

In figure 7 is illustrated the injection pipe as described above.

In table 1 is illustrated the outcome of tests made with the system. Column A represents the liquid flow in combination with the gas volume introduced into the liquid flow by means of the venturi created in the mixing chamber 20 (see figure 6). The volume which the pump on itself is able to inject into the tank is represented by column B. As is evident the volume, when combining gas with liquid, is substantially larger than only liquid at the same energy consumption and therefore indicates that the pump's effectiveness is much increased when adding gas to the liquid such that a larger force creating the turbulence inside the tank is created with a smaller pump than what would be possible with the pump without gas mixture on its own.

The invention therefore provides more volume with less energy which in turn creates better turbulence/circulation inside the tank and at the same time by passing the basic material together with the liquid through a chopper or cutter pump and the venturi device creates hydrolysis such that the bacteria inside the tank will have a much larger surface to attack thereby producing more biogas, faster. Furthermore, tests indicate clearly that the yield is increased due to recirculation of the biogas through the tank as is made possible by the gas mix installation according to the present invention.

Above, the invention has been described with reference to specific embodiments but the invention is only to be limited by the scope of the appended claims.

## Claims

1. Gas mix installation (5) for use in connection with tanks for biogas reactors (1) wherein the gas mix installation comprises:
- a tank (1)
- a main pump (8) having respectively a suction side and an injection side, where said pump (8) is arranged externally of the tank (1) where the pump (8) is a shredder type pump, comprising means for cutting, chopping or shredding any solids in the liquid;
- a liquid suction pipe (6) connecting the interior of the tank (1) to the main pump's (8) suction side;
- an injector pipe (7) connecting the interior of the tank to the pump's injection side wherein the injector pipe, at least the part of the pipe which in use projects into the tank, is arranged at an angle relative to the side of the wall of the tank, where said angle is selected between 15° and 40°.;
- a gas pipe (10) connected between the injector pipe (7) and a source of biogas (9); where said gas pipe (10) is connected to the injector pipe (7) externally of the tank (1).

2. Gas mix installation according to claim 1 wherein the gas pipes' inlet in the injector pipe is in a venturi device arranged in the injector pipe.

3. Gas mix installation according to claim 1 or 2 wherein the injector pipe upstream from the connection to the gas pipe is provided with a branch pipe, which branch pipe is provided with a secondary injector pipe.

4. Gas mix installation according to claim 1 or 2 wherein the diameter of the injector pipe at least on a section of said pipe has an interior diameter of 60 - 100 mm.

5. Gas mix installation according to claim 1 wherein in use the injector pipe is arranged at an angle relative to the wall of the tank, which is different to the angle at which the suction pipe or at least the the part of the suction pipe which projects into the tank, is arranged relative to the wall of the tank.

6. Gas mix installation according to claim 1 or 2 wherein the suction pipe in use is arranged below the injection pipe.

7. Gas mix installation according to claim 1 wherein the injector pipe has a longitudinal axis, and where said injector pipe comprises a nozzle having an opening where said opening has a first diameter, connecting to a mixing chamber where said mixing chamber in at least one cross-section orthogonal to the longitudinal axis has a diameter larger than the first diameter, where said mixing chamber connects to an ejector pipe, which in turn is connected to a transition pipe section, which section is conical, having its smallest diameter adjacent the ejector pipe, and where optionally a diffuser is arranged in the opposite end of the transition pipe.

8. Method of increasing the gas yield from a biogas reactor tank, wherein one or more gas mix installations according to any of claims 1 to 7 is installed at a predetermined distance from the bottom of the reactor tank;
where the injector pipes are arranged to inject a gas/liquid mix into the tank at an oblique angle relative to the wall of the reactor tank, whereby the liquid in the tank is stirred;
where the gas pipe uses the reservoir of biogas above the surface of the liquid, inside the tank as a source of gas, allowing the gas to percolate up through the liquid.

9. Method according to claim 8, wherein the under-pressure in the suction pipe and the over-pressure in the injection pipe causes the liquid inside the tank to rotate in a horizontal plane and circulate in a vertical plane.

10. Method according to claim 8, wherein a plurality of gas mix installations are arranged along the periphery of the bioreactor, in one or more levels relative to the tank's bottom.

11. Method according to any of claims 8 to 10, using a gas mix installation according to any of claims 1 to 7, wherein means are provided for injecting an enzyme into the liquid in the suction pipe, or injecting the enzyme in the venturi device.

## Patentansprüche

1. Gasmischanlage (5) zur Verwendung in Verbindung mit Behältern für Biogasreaktoren (1), wobei die Gasmischanlage Folgendes umfasst:
- einen Behälter (1)
- eine Hauptpumpe (8), die jeweils eine Saugseite und eine Spritzseite aufweist, wobei die Pumpe (8) außerhalb des Behälters (1) angeordnet ist, wobei die Pumpe (8) ein Pumpentyp mit Zerkleinerer ist, die Mittel zum Zerschneiden, Häckseln oder Zerkleinern von Feststoffen in der Flüssigkeit umfasst;
- eine Flüssigkeitssaugleitung (6), die das Innere des Behälters (1) mit der Saugseite der Hauptpumpe (8) verbindet;
- eine Einspritzleitung (7), die das Innere des Behälters mit der Spritzseite der Pumpe verbindet, wobei die Einspritzleitung, zumindest der Teil der Leitung, der im Betrieb in den Behälter hineinragt, in einem Winkel im Verhältnis zu der Seite der Wand des Behälters angeordnet ist, wobei der Winkel zwischen 15° und 40° ausgewählt ist;
- eine Gasleitung (10), die zwischen der Einspritzleitung (7) und einer Quelle von Biogas (9) angeschlossen ist;
wobei die Gasleitung (10) mit der Einspritzleitung (7) außerhalb des Behälters (1) verbunden ist.

2. Gasmischanlage nach Anspruch 1, wobei die Zuführung der Gasleitung in die Einspritzleitung in einer Venturi-Vorrichtung erfolgt, die in der Einspritzleitung angeordnet ist.

3. Gasmischanlage nach Anspruch 1 oder 2, wobei die Einspritzleitung vor der Verbindung zur Gasleitung mit einer Abzweigleitung versehen ist, wobei die Abzweigleitung mit einer sekundären Einspritzleitung versehen ist.

4. Gasmischanlage nach Anspruch 1 oder 2, wobei der Durchmesser der Einspritzleitung mindestens auf einem Abschnitt der Leitung einen Innendurchmesser von 60 - 100 mm aufweist.

5. Gasmischanlage nach Anspruch 1, wobei im Betrieb die Einspritzleitung in einem Winkel im Verhältnis zur Wand des Behälters angeordnet ist, der sich von dem Winkel, in dem die Saugleitung oder zumindest der Teil der Saugleitung, der in den Behälter hineinragt, im Verhältnis zur Wand des Behälters angeordnet ist, unterscheidet.

6. Gasmischanlage nach Anspruch 1 oder 2, wobei die Saugleitung im Betrieb unter der Einspritzleitung angeordnet ist.

7. Gasmischanlage nach Anspruch 1, wobei die Einspritzleitung eine Längsachse aufweist und wobei die Einspritzleitung eine Düse umfasst, die eine Öffnung aufweist, wobei die Öffnung einen ersten Durchmesser aufweist, und an eine Mischkammer anschließt, wobei die Mischkammer in mindestens einem Querschnitt, der rechtwinklig zur Längsachse ist, einen Durchmesser aufweist, der größer als der erste Durchmesser ist, wobei die Mischkammer an eine Auswurfleitung anschließt, die wiederum an einen Übergangsleitungsabschnitt anschließt, wobei der Abschnitt konisch ist und seinen kleinsten Durchmesser an die Auswurfleitung angrenzend aufweist und wobei optional ein Diffusor am entgegengesetzten Ende der Übergangsleitung angeordnet ist.

8. Verfahren zum Erhöhen der Gasausbeute aus einem Biogasreaktorbehälter, wobei eine oder mehrere Gasmischanlagen nach einem der Ansprüche 1 bis 7 in einem vorbestimmten Abstand vom Boden des Reaktorbehälters eingebaut ist/sind;
wobei die Einspritzleitungen angeordnet sind, um ein Gas-/Flüssigkeitsgemisch in den Behälter in einem schrägen Winkel im Verhältnis zur Wand des Reaktorbehälters einzuspritzen, wobei die Flüssigkeit im Behälter gerührt wird;
wobei die Gasleitung das Biogasreservoir über der Oberfläche der Flüssigkeit innerhalb des Behälters als eine Gasquelle nutzt, was es dem Gas ermöglicht, durch die Flüssigkeit nach oben zu dringen.

9. Verfahren nach Anspruch 8, wobei der Unterdruck in der Saugleitung und der Überdruck in der Einspritzleitung die Flüssigkeit innerhalb des Behälters dazu bringen, in einer horizontalen Ebene zu rotieren und in einer vertikalen Ebene zu zirkulieren.

10. Verfahren nach Anspruch 8, wobei eine Vielzahl von Gasmischanlagen entlang dem Umfang des Bioreaktors in einer oder mehreren Höhen im Verhältnis zum Boden des Behälters angeordnet sind.

11. Verfahren nach einem der Ansprüche 8 bis 10, das eine Gasmischanlage nach einem der Ansprüche 1 bis 7 verwendet, wobei Mittel vorgesehen sind, um ein Enzym in die Flüssigkeit in der Saugleitung einzuspritzen oder das Enzym in die Venturi-Vorrichtung einzuspritzen.

## Revendications

1. Installation de mélange de gaz (5) pour une utilisation en connexion avec des cuves pour réacteurs de biogaz (1) dans laquelle l'installation de mélange de gaz comprend :
- une cuve (1)
- une pompe principale (8) ayant respectivement un côté aspiration et un côté injection, où ladite pompe (8) est agencée à l'extérieur de la cuve (1) où la pompe (8) est une pompe de type déchiqueteuse, comprenant des moyens pour découper, hacher ou déchiqueter n'importe quel solide dans le liquide ;
- un tuyau d'aspiration de liquide (6) connectant l'intérieur de la cuve (1) au côté aspiration de la pompe principale (8) ;
- un tuyau d'injecteur (7) connectant l'intérieur de la cuve au côté injection de la pompe dans laquelle le tuyau d'injecteur, au moins la partie du tuyau qui est en utilisation fait saillie dans la cuve, est agencé à un angle par rapport au côté de la paroi de la cuve, où ledit angle est sélectionné entre 15° et 40° ;
- un tuyau de gaz (10) connecté entre le tuyau d'injecteur (7) et une source de biogaz (9) ;
où ledit tuyau de gaz (10) est connecté au tuyau d'injecteur (7) à l'extérieur de la cuve (1).

2. Installation de mélange de gaz selon la revendication 1 dans laquelle l'entrée du tuyau de gaz dans le tuyau d'injecteur est dans un dispositif venturi agencé dans le tuyau d'injecteur.

3. Installation de mélange de gaz selon la revendication 1 ou 2 dans laquelle le tuyau d'injecteur en amont de la connexion au tuyau de gaz est pourvu d'un tuyau de ramification, lequel tuyau de ramification est pourvu d'un tuyau d'injecteur secondaire.

4. Installation de mélange de gaz selon la revendication 1 ou 2 dans laquelle le diamètre du tuyau d'injecteur au moins sur une section dudit tuyau présente un diamètre interne de 60 à 100 mm.

5. Installation de mélange de gaz selon la revendication 1 dans laquelle en utilisation le tuyau d'injecteur est agencé à un angle par rapport à la paroi de la cuve, qui est différent de l'angle auquel le tuyau d'aspiration ou au moins la partie du tuyau d'aspiration qui fait saillie dans la cuve, est agencé par rapport à la paroi de la cuve.

6. Installation de mélange de gaz selon la revendication 1 ou 2 dans laquelle le tuyau d'aspiration en utilisation est agencé en dessous du tuyau d'injection.

7. Installation de mélange de gaz selon la revendication 1 dans laquelle le tuyau d'injecteur possède un axe longitudinal, et où ledit tuyau d'injecteur comprend une buse ayant une ouverture où ladite ouverture présente un premier diamètre, se connectant à une chambre de mélange où ladite chambre de mélange dans au moins une section transversale orthogonale à l'axe longitudinal présente un diamètre supérieur au premier diamètre, où ladite chambre de mélange se connecte à un tuyau d'éjecteur, qui à son tour est connecté à une section de tuyau de transition, laquelle section est conique, ayant son plus petit diamètre adjacent au tuyau d'éjecteur, et où facultativement un diffuseur est agencé à l'extrémité opposée du tuyau de transition.

8. Procédé d'augmentation du rendement en gaz provenant d'une cuve de réacteur de biogaz, dans lequel une ou plusieurs installations de mélange de gaz selon l'une quelconque des revendications 1 à 7 sont installées à une distance prédéterminée par rapport au fond de la cuve de réacteur ;
où les tuyaux d'injecteur sont agencés pour injecter un mélange gaz/liquide dans la cuve à un angle oblique par rapport à la paroi de la cuve de réacteur, moyennant quoi le liquide dans la cuve est agité ;
où le tuyau de gaz utilise le réservoir de biogaz au-dessus de la surface du liquide, à l'intérieur de la cuve comme source de gaz, en permettant au gaz de percoler à travers le liquide.

9. Procédé selon la revendication 8, dans lequel la sous-pression dans le tuyau d'aspiration et la surpression dans le tuyau d'injection amènent le liquide à l'intérieur de la cuve à tourner dans un plan horizontal et à circuler dans un plan vertical.

10. Procédé selon la revendication 8, dans lequel une pluralité d'installations de mélange de gaz est agencée le long de la périphérie du bioréacteur, à un ou plusieurs niveaux par rapport au fond de la cuve.

11. Procédé selon l'une quelconque des revendications 8 à 10, utilisant une installation de mélange de gaz selon l'une quelconque des revendications 1 à 7, dans lequel des moyens sont fournis pour injecter une enzyme dans le liquide dans le tuyau d'aspiration, ou injecter l'enzyme dans le dispositif venturi.
